# EUROPÄISCHE PATENTSCHRIFT

(11) **EP 3 389 790 B1**
(45) Veröffentlichungstag und Bekanntmachung des Hinweises auf die Patenterteilung: **03.02.2021**
(21) Anmeldenummer: 16822413.7
(22) Anmeldetag: 15.12.2016
(51) Int. Cl.: A61Q 5/08, A61Q 5/10, A61K 8/22, A61K 8/20, A61K 8/23, A61K 8/24, A61K 8/365, A61K 8/36

(54) **OXIDATIONSMITTELZUBEREITUNGEN**
OXIDANT PREPARATION
PRÉPARATION D'OXYDANT

(30) Priorität: 18.12.2015 DE 102015225895
(43) Veröffentlichungstag der Anmeldung: 24.10.2018
(73) Patentinhaber: Henkel AG & Co. KGaA, 40589 Düsseldorf (DE)
(72) Erfinder: GROSJACQUES, Camille, 20255 Hamburg (DE); HAGENOW, Susanne, 20359 Hamburg (DE); KERL, Sylvia, 22763 Hamburg (DE); MANNECK, Hartmut, 23858 Barnitz (DE); KLEEN-FEHRES, Astrid, 20457 Hamburg (DE)
(86) Internationale Anmeldenummer: PCT/EP2016/081173
(87) Internationale Veröffentlichungsnummer: WO 2017/102936

(56) Entgegenhaltungen:
- EP-A1- 2 471 504
- WO-A1-2005/067874
- WO-A1-2015/090882
- WO-A2-2014/174230
- DE-A1-102008 062 239
- DE-A1-102011 082 918
- JP-A- 2006 290 857
- US-A1- 2006 198 803

## Beschreibung

Die vorliegende Erfindung liegt auf dem Gebiet der Kosmetik und betrifft spezielle Oxidationsmittelzubereitungen für die oxidative Farbveränderung von keratinischen Fasern, insbesondere menschlichen Haaren. Weitere Gegenstände der vorliegenden Erfindung sind Verfahren zur oxidativen Farbveränderung von menschlichen Haaren, bei welchen die spezielle Oxidationsmittelzubereitung zum Einsatz kommt.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln untereinander die eigentlichen Farbstoffe ausbilden. Oxidationsfärbemittel zeichnen sich durch lang anhaltende Färbeergebnisse aus.

Kurz vor der Anwendung eines oxidativen Färbemittels wird üblicherweise eine erste Zubereitung (Oxidationsmittelzubereitung), welche als Oxidationsmittel Wasserstoffperoxid enthält, mit einer zweiten Zubereitung (Farbcreme oder Färbezubereitung), welche die Oxidationsfarbstoffvorprodukte enthält, vermischt. Mit diesem anwendungsbereiten oxidative Färbemittel werden dann die keratinischen Fasern gefärbt. (siehe z.B. EP2471504 A1 oder DE102008 062239 A1 oder DE102011082918 A1)

Im Friseurbereich besonders häufig verwendet werden die Level 3 Colorationen. Bei den Level 3 Colorationen handelt es sich um oxidative Färbemittel, die sich durch eine besonders gute Haltbarkeit und eine besonders hohes Grauabdeckungsvermögen auszeichnen.

Erzielt werden können diese gute Haltbarkeit und die guten Grauabdeckung durch einen relativ hohen Ammoniak-Gehalt in den Colorationen, der zu einer starken Quellung des Haares und hierdurch bedingt zu einer hohen Diffusionsrate der Oxidationsfarbstoffvorprodukte in das Haar hinein führt. Bei dunklen Nuancen der Level 3 Colorationen ist zudem der Gehalt an Oxidationsfarbstoffvorprodukten vergleichsweise hoch.

Verbunden mit diesem hohen Ammoniak-Gehalt ist jedoch ebenfalls auch eine hohe Haarschädigung.

Im Heimanwenderbereich kann der Anwender, der diese hohe Haarschädigung nicht bei jeder Färbung nicht in Kauf nehmen möchte, auf Level 2 Produkte ausweichen. Bei den Level 2 Produkten handelt es sich ebenfalls um oxidative Färbemittel, jedoch ist deren Ammoniak-Gehalt geringer, oder aber es werden anstatt Ammoniak alternative, weniger stark quellende Alkalisierungsmittel eingesetzt. Im Heimanwenderbereich sind Level 3 und Level 2 Produkte separat verpackt und werden als getrennte Produkte vertrieben, so dass der Anwender sich entweder ein Level 3 oder ein Level 2 Produkt aussuchen und applizieren kann.

Im Friseurbereich stellt der Friseur seinem Kunden eine viel umfangreichere Nuancenpalette zur Verfügung. Eine komplette Level 3 Färbeserie umfasst so eine Palette verschiedenster Farbcremes, welche jeweils kurz vor der Anwendung mit der üblichen Level 3 Oxidationsmittelzubereitung vermischt werden. Aus Kapazitäts- und Lagergründen wird der Friseur es daher vermeiden, sowohl eine vollständige Nuancenpalette für Level 3 Produkte und für Level 2 Produkte vorrätig zu halten.

Es war daher die Aufgabe der vorliegenden Erfindung, dem Friseur einen flexibel und leicht anwendbare Möglichkeit oder Methode zur Verfügung zu stellen, die es dem Friseur erlaubt, aus einem Level 3 Färbeprodukt ein Level 2 Produkt zu erzeugen.

Im Zuge der zu dieser Erfindung führenden Arbeiten hat sich nun herausgestellt, dass der Einsatz einer speziellen Oxidationsmittelzubereitung es dem Friseur erlaubt, aus den üblichen, zu einer Level 3 Färbung gehörenden Nuancenpalette ein Level 2 Färbesystem zu generieren, ohne dass auf dem gefärbten Haar eine Farbverschiebung auftritt.

Durch Abmischung der speziellen, erfindungsgemäßen Oxidationsmittelzubereitung mit den üblichen, zum Level 3 Färbesystem gehörenden Farbcremes kann der Friseur auf diese Weise ein anwendungsbereites Färbemittel erzeugen, dessen Alkalität gezielt reduziert wurde und das genau auf Haare mit höherem Schädigungsgrad abgestimmt ist.

Der Friseur kann auf diese Weise seinem Kunden, dessen Haare bislang immer mit einem bestimmten Level 3 Produkt gefärbt worden waren, die gewünschte Farbnuance mit genau demselben Nuancenausfall zur Verfügung stellen. Aufgrund des Einsatzes der speziellen Oxidationsmittelzubereitung ist der Gehalt an Alkalisierungsmitteln in der Anwendungsmischung jedoch gezielt reduziert und die Anwendungsmischung so an die durch wiederholte Färbungen inzwischen stärker geschädigten Haare des Kunden angepasst.

Ein erster Gegenstand der vorliegenden Erfindung ist diese spezielle Oxidationsmittelzubereitung.

Ein Gegenstand der vorliegenden Erfindung ist eine Oxidationsmittelzubereitung zur oxidativen Farbveränderung von keratinischen Fasern, insbesondere menschlichen Haaren, enthaltend in einem wässrigen kosmetischen Träger
(A) Wasserstoffperoxid und
(B) Äpfelsäure,
wobei die Oxidationsmittelzubereitung einen pH-Wert von 0,5 bis 3,0 besitzt.

Die erfindungsgemäße Oxidationsmittelzubereitung ist speziell auf den Einsatz im Friseurbereich zugeschnitten. Kurz vor der Anwendung vermischt der Friseur die Oxidationsmittelzubereitung mit einer zweiten Zubereitung, welche die Oxidationsfarbstoffvorprodukte und/oder Alkalisierungsmittel enthält. Durch das Vermischen der Oxidationsmittelzubereitung und die zweiten Zubereitung wird das anwendungsbereite Mittel zur oxidativen Farbveränderung der keratinischen Fasern erhalten.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Bei dem erfindungsgemäßen Produkt handelt es sich um ein Produkt für die oxidative Farbveränderung von keratinischen Fasern, d.h. um ein Produkt, dass auf dem menschlichen Kopf angewendet wird, um eine oxidative Färbung, eine Aufhellung, eine Blondierung, eine Bleiche oder eine Nuancierung der Haare zu erzielen. Unter Nuancierung wird in diesem Zusammenhang eine Färbung verstanden, bei welcher das Farbresultat heller als die Ausgangshaarfarbe ist.

Die erfindungsgemäße Oxidationsmittelzubereitung enthält die erfindungswesentlichen Bestandteile jeweils in einem wässrigen kosmetischen Träger, bevorzugt in einem geeigneten wässrigen oder wässrig-alkoholischen Träger. Zum Zwecke der oxidativen Färbänderung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein.

Die erfindungsgemäße Oxidationsmittelzubereitung enthält als Oxidationsmittel (A) Wasserstoffperoxid.

Die Konzentration des Wasserstoffperoxids (A) in der Oxidationsmittelzubereitung wird einerseits von den gesetzlichen Vorgaben und andererseits von dem gewünschten Aufhelleffekt bestimmt; vorzugsweise werden 0,5 bis 20,0 Gew.-%ige Lösungen in Wasser verwendet. Erfindungsgemäß bevorzugte Oxidationsmittelzubereitungen sind dadurch gekennzeichnet, dass sie, bezogen auf ihr Gesamtgewicht, Wasserstoffperoxid (A) in einer Menge von 0,5 bis 12,5 Gew.-%, bevorzugt von 1,0 bis 9,0 Gew.-%, weiter bevorzugt von 1,5 bis 6,5 Gew.-% und ganz besonders bevorzugt von 2,0 bis 4,5 Gew-% enthalten. Alle Angaben in Gew.-% sind hierbei auf das Gewicht des in der Oxidationsmittelzubereitung enthaltenen Wasserstoffperoxids bezogen (berechnet als 100 %iges H2O2), das zum Gesamtgewicht der Oxidationsmittelzubereitung in Relation gesetzt wird.

In einer besonders bevorzugten Ausführungsform ist eine erfindungsgemäße Oxidationsmittelzubereitung daher dadurch gekennzeichnet, dass sie - bezogen auf ihr Gesamtgewicht - Wasserstoffperoxid (A) in einer Menge von 0,5 bis 12,5 Gew.-%, bevorzugt von 1,0 bis 9,0 Gew.-%, weiter bevorzugt von 1,5 bis 6,5 Gew.-% und ganz besonders bevorzugt von 2,0 bis 4,5 Gew-% enthält.

Zentrale und erfindungswesentliche Merkmale der Oxidationsmittelzubereitung sind (B) ihr Gehalt an bestimmten Säuren und ihr besonders niedriger pH-Wert.

Die Oxidationsmittelzubereitung ist erfindungsgemäß dadurch gekennzeichnet, dass sie Äpfelsäure enthält.

Äpfelsäure wird alternativ auch als 2-Hydroxybernsteinsäure oder 2-Hydroxybutandisäure bezeichnet. Äfpelsäure liegt in Form zweier Enantiomere vor, (D-Äpfelsäure (CAS-Nr. 636-61-3) und L-Äpfelsäure (CAS-Nr. 97-67-6)), die beide, sowohl in ihrer isolierten Form als auch als Gemisch, von der Erfindung mit umfasst sind.

Das zweite erfindungswesentliche Merkmal der Oxidationsmittelzubereitung ist ihr besonders niedriger pH-Wert, der im Bereich von 0,5 bis 3,0 liegt.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.

Eine besonders effektive Reduktion des Alkalisierungsmittelgehaltes in der Farbcreme (bzw. Färbezubereitung) eines Level 3 Produktes ist möglich, wenn die Oxidationsmittelzubereitung einen pH-Wert im Bereich von 0,5 bis 2,6, bevorzugt von 0,5 bis 2,4, weiter bevorzugt von 0,5 bis 2,2, noch weiter bevorzugt von 0,5 bis 2,0, noch weiter bevorzugt von 0,5 bis 1,8 und ganz besonders bevorzugt von 0,5 bis 1,6 besitzt.

In einer weiteren ganz besonders bevorzugten Ausführungsform ist eine erfindungsgemäße Oxidationsmittelzubereitung daher dadurch gekennzeichnet, dass sie einen pH-Wert im Bereich von 0,5 bis 2,6, bevorzugt von 0,5 bis 2,4, weiter bevorzugt von 0,5 bis 2,2, noch weiter bevorzugt von 0,5 bis 2,0, noch weiter bevorzugt von 0,5 bis 1,8 und ganz besonders bevorzugt von 0,5 bis 1,6 besitzt.

Um den pH-Wert auch während der Lagerung stabil im gewünschten niedrigen Bereich zu halten, werden der Oxidationsmittelzubereitung bevorzugt auch noch eine oder weitere zusätzliche Säuren zugesetzt, die von den Säuren aus der Gruppe (B) verschieden sind. Besonders bevorzugt ist in diesem Zusammenhang der Zusatz einer oder mehrerer Säuren aus einer weiteren Gruppe (C), die neben ihrer acidifizierenden Wirkung auch noch in der Lage sind, das Wasserstoffperoxid zu stabilisieren. Besonders bevorzugte zusätzliche, stabilisierend wirkende Säuren können ausgewählt werden aus der Gruppe (C) aus Etidronsäure, Benzoesäure, Pyridin-2,6-dicarbonsäure, Phthalsäure und/oder Salicylsäure.

In einer weiteren ganz besonders bevorzugten Ausführungsform ist eine erfindungsgemäße Oxidationsmittelzubereitung daher dadurch gekennzeichnet, dass sie zusätzlich
(C) eine oder mehrere Säuren aus der Gruppe aus Etidronsäure, Benzoesäure, Pyridin-2,6-dicarbonsäure, Phthalsäure und/oder Salicylsäure enthält.

Etidronsäure wird alternativ auch als 1-Hydroxyethan-1,1-diphosphonsäure bezeichnet.

Welche Mengen der Äpfelsäure in der erfindungsgemäßen Oxidationsmittelzubereitung eingesetzt werden, hängt zum einen von der Säurestärke der Äpfelsäure und zum anderen auch von weiteren in der Oxidationsmittelzubereitung vorhandenen, ggf. alkalisch reagierenden Inhaltstoffen ab. Es ist bevorzugt, wenn die Oxidationsmittelzubereitung eine oder mehrere Säuren aus der Gruppe (B) in einer Gesamtmenge von 0,5 bis 15,0 Gew.-%, bevorzugt von 0,5 bis 10,0 Gew.-%, weiter bevorzugt von 0,5 bis 8,0 Gew.-% und ganz besonders bevorzugt von 1,0 bis 6,0 Gew.-% enthält. Hierbei sind alle Mengenangaben in Gew.-% auf das Gesamtgewicht aller in der Oxidationsmittelzubereitung enthaltenen Säuren aus der Gruppe (B) bezogen, das zum Gesamtgewicht der Oxidationsmittelzubereitung in Relation gesetzt wird.
In einer weiteren ganz besonders bevorzugten Ausführungsform ist eine erfindungsgemäße Oxidationsmittelzubereitung daher dadurch gekennzeichnet, dass sie - bezogen auf ihr Gesamtgewicht - eine oder mehrere Säuren aus der Gruppe (B) in einer Gesamtmenge von 0,5 bis 15,0 Gew.-%, bevorzugt von 0,5 bis 10,0 Gew.-%, weiter bevorzugt von 0,5 bis 8,0 Gew.-% und ganz besonders bevorzugt von 1,0 bis 6,0 Gew.-% enthält.

Auch die Einsatzmenge der Säuren aus der Gruppe (C) in der erfindungsgemäßen Oxidationsmittelzubereitung hängt zum einen von der Säurestärke der jeweiligen Säure und zum anderen auch von weiteren in der Oxidationsmittelzubereitung vorhandenen, ggf. alkalisch reagierenden Inhaltstoffen ab. Es ist bevorzugt, wenn die Oxidationsmittelzubereitung eine oder mehrere Säuren aus der Gruppe (C) in einer Gesamtmenge von 0,05 bis 10,0 Gew.-%, bevorzugt von 0,1 bis 8,0 Gew.-%, weiter bevorzugt von 0,15 bis 6,0 Gew.-% und ganz besonders bevorzugt von 0,17 bis 3,0 Gew.-% enthält. Hierbei sind alle Mengenangaben in Gew.-% auf das Gesamtgewicht aller in der Oxidationsmittelzubereitung enthaltenen Säuren aus der Gruppe (C) bezogen, das zum Gesamtgewicht der Oxidationsmittelzubereitung in Relation gesetzt wird.

In einer weiteren ganz besonders bevorzugten Ausführungsform ist eine erfindungsgemäße Oxidationsmittelzubereitung daher dadurch gekennzeichnet, dass sie - bezogen auf ihr Gesamtgewicht - eine oder mehrere Säuren aus der Gruppe (C) in einer Gesamtmenge von 0,05 bis 10,0 Gew.-%, bevorzugt von 0,1 bis 8,0 Gew.-%, weiter bevorzugt von 0,15 bis 6,0 Gew.-% und ganz besonders bevorzugt von 0,17 bis 3,0 Gew.-% enthält.

Um eine schnelle und gute Vermischbarkeit der Oxidationsmittelzubereitung mit der Färbezubereitung
zu gewährleisten, ist die Oxidationsmittelzubereitung besonders bevorzugt mit einem oder mehreren Fettbestandteilen verdickt und liegt somit in Form einer Emulsion vor.

In einer besonders bevorzugten Ausführungsform ist die erfindungsgemäße Oxidationsmittelzubereitung dadurch gekennzeichnet, dass sie - bezogen auf ihr Gesamtgewicht - einen oder mehrere Fettbestandteile in einer Gesamtmenge 1,0 bis 10,0 Gew.-%, bevorzugt von 1,5 bis 7,5 Gew.-%, weiter bevorzugt von 2,0 bis 7,0 Gew.-% und ganz besonders bevorzugt von 2,5 bis 6,5 Gew.-% enthält.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 8 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Als bevorzugte Fettbestandteile werden in diesem Zusammenhang die Bestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den C₁₂-C₃₀-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13*Z*)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Unter einem C₁₂-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₁₂-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₁₂-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₁₂-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)-Tetracos-15-ensäure].

Unter einem C₁₂-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Geeignete Paraffinöle sind insbesondere flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Bevorzugte Fettbestandteile sind ausgewählt aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe. Die C₁₂-C₃₀-Fettalkohole und/oder die Kohlenwasserstoffe sind bevorzugte Fettbestandteile. Ganz besonders bevorzugte Fettbestandteile sind die C₁₂-C₃₀-Fettalkohole.

In einer besonders bevorzugten Ausführungsform ist die erfindungsgemäße Oxidationsmittelzubereitung dadurch gekennzeichnet, dass sie - bezogen auf ihr Gesamtgewicht - einen oder mehrere C₁₂-C₃₀-Fettalkohole in einer Gesamtmenge 1,0 bis 10,0 Gew.-%, bevorzugt von 1,5 bis 7,5 Gew.-%, weiter bevorzugt von 2,0 bis 7,0 Gew.-% und ganz besonders bevorzugt von 2,5 bis 6,5 Gew.-% enthält.

Die erfindungsgemäße, zuvor beschriebene Oxidationsmittelkomponente kann vom Friseur in einem Verfahren zur oxidativen Farbveränderung von menschlichen Haaren eingesetzt werden.

Ein zweiter Gegenstand der vorliegenden Erfindung ist insbesondere ein Verfahren zur oxidativen Farbveränderung von menschlichen Haaren, umfassend die folgenden Schritte in der angegebenen Reihenfolge
A) Vermischen einer ersten Komponente (K1) mit einer zweiten Komponente (K2) unter Erhalt einer Mischung (M1),
B) Applizieren der Mischung (M1) auf die Haare,
C) Einwirkenlassen der Mischung (M1) für einen Zeitraum von 30 Sekunden bis 45 Minuten,
D) Ausspülen der Mischung (M1) aus den Haaren,
wobei
- die erste Komponente (K1) eine Oxidationsmittelzubereitung ist, wie sie im Detail bei Beschreibung des ersten Erfindungsgegenstands offenbart ist,
- die zweite Komponente (K2) eine Färbezubereitung ist, die mindestens ein Oxidationsfarbstoffvorprodukt und/oder mindestens ein Alkalisierungsmittel enthält.

In dem erfindungsgemäßen Verfahren wird im Schritt A) die Oxidationsmittelzubereitung (die erste Komponente (K1)) mit einer Färbezubereitung (der zweiten Komponente (K2)) vermischt. Bei der Färbezubereitung (K2) handelt es sich beispielsweise um eine üblicherweise vorhandene Level 3 Farbcreme, die mindestens ein Oxidationsfarbstoffvorprodukt und/oder mindestens ein Alkalisierungsmittel enthält. Bevorzugt enthält die zweite Komponente (K1) mindestens ein Oxidationsfarbstoffvorprodukt vom Entwicklertyp und mindestens ein Oxidationsfarbstoffvorprodukt vom Kupplertyp. Die Vermischung von (K1) und (K2) kann beispielsweise durch Verrühren oder Verschütteln erfolgen.

Nach Herstellung der Mischung (M1) wird diese in Schritt B) auf die Haare appliziert, wobei die Mischung (M1) entweder auf den gesamten Bereich der zu färbenden Haare oder nur auf bestimmte Haarpartien (wie beispielsweise den Haaransatz oder die Haarlängen/Spitzen) aufgetragen werden kann.

Nach dem Auftragen wird die Mischung (M1) dann in Schritt C) für einen Zeitraum von 30 Sekunden bis 45 Minuten auf die Haare einwirken gelassen. Hierbei ist es möglich und erfindungsgemäß, die Mischung (M1) für einen bestimmten Zeitraum auf allen Partien des Haares zu belassen. In einer weiteren Ausführungsform ist es jedoch ebenfalls möglich, die Einwirkdauer für bestimmte Haarpartien verschieden zu wählen, so dass beispielsweise die Einwirkdauer im Bereich das Haaransatzes länger ist als die Einwirkdauer im Bereich der geschädigten Spitzen.

Nach dem Einwirkenlassen wird die Mischung (M1) dann in Schritt D) wieder aus den Haaren ausgespült. Das Ausspülen kann entweder nur mit Wasser oder aber unter Zuhilfenahme eines Shampoos erfolgen.

Die Schritte A) bis D) sind die Schritte eines einzigen Färbeverfahrens, d.h. alle Schritte werden erfindungsgemäß während eines Färbevorgangs, d.h. innerhalb eines Zeitraumes von maximal 6 Stunden, bevorzugt innerhalb eines Zeitraums von maximal 3 Stunden, durchgeführt.

Im erfindungsgemäßen Verfahren ist auch die Reihenfolge der Schritte festgelegt und findet in der Reihenfolge A), gefolgt von B), gefolgt von C), gefolgt von D) statt.

Bevorzugt werden die Komponenten (K1) und (K2) im erfindungsgemäßen Verfahren in bestimmten Mengenbereichen miteinander vermischt. Die erste Komponente (K1) und die zweite Komponente (K2) können beispielsweise in einem Mengenverhältnis von 3:1 bis 1:3, bevorzugt von 2:1 bis 2:1, miteinander vermischt werden.

In einer weiteren ganz besonders bevorzugten Ausführungsform ist das erfindungsgemäße Verfahren zur oxidativen Farbveränderung von menschlichen Haaren dadurch gekennzeichnet, dass
- die erste Komponente (K1) und die zweite Komponente (K2) in einem Gewichtsverhältnis von 3:1 bis 1:3, bevorzugt von 2:1 bis 1:2 und besonders bevorzugt von 1,5:1 bis 1:1,5 miteinander vermischt werden.

Beispiel: Wenn 150 g der Oxidationsmittelzubereitung (K1) und 100 g der Färbezubereitung (K2) miteinander vermischt werden, so beträgt das Gewichtsverhältnis 1,5:1.

Zur Verminderung des pH-Wertes der Farbcreme (K2) im Vergleich zu einer üblichen Level 3 Coloration wird diese mit einer speziellen, auf einen besonders niedrigen pH-Wert eingestellten Oxidationsmittelzubereitung (K1) vermischt.

Bei der Farbcreme (K2) handelt es sich um eine Zubereitung, die bevorzugt mindestens ein Oxidationsfarbstoffvorprodukt vom Entwicklertyp und mindestens ein Oxidationsfarbstoffvorprodukt vom Kupplertyp enthält.

Bevorzugte zusätzliche Oxidationsfarbstoffvorprodukte vom Entwickler-Typ können ausgewählt werden aus der Gruppe, die gebildet wird aus 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetra-oxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

In einer weiteren ganz besonders bevorzugten Ausführungsform ist das erfindungsgemäße Verfahren zur oxidativen Farbveränderung von menschlichen Haaren dadurch gekennzeichnet, dass dass die zweite Komponente (K2) mindestens ein Oxidationsfarbstoffvorprodukt aus der Gruppe aus p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4-Aminophenol, 4-Amino-3-methylphenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin und/oder deren physiologisch verträglichen Salzen enthält.

Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Dihydroxybenzol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

Bevorzugte Oxidationsfarbsotffvorprodukte vom Kupplertyp können ausgewählt werden aus der Gruppe aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin und/oder 7-Hydroxyindolin und deren physiologisch verträglichen Salzen.

Weiterhin kann die Komponente (K2) zusätzlich auch noch einen oder mehrere direktziehende Farbstoffe enthalten.

Der pH-Wert der Komponente (K2) ist alkalisch eingestellt. Die zur Einstellung der bevorzugten pH-Werte erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)-Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.

Bei der zweiten Komponente (K2) handelt es sich um eine Farbcreme, wie sie in einem üblichen Level 3 Colorationsprodukt üblich ist. Zur Erzeugung von Färbungen mit hoher Haltbarkeit und guter Grauabdeckungen enthalten die Farbcremes daher eine verhältnismäßig hohe Menge an Ammoniak.

Im erfindungsgemäßen Verfahren wird durch Vermischen der Farbcreme (K2) mit der Oxidationsmittelzubereitung (K1) dieser Ammoniak-Gehalt in definierter und reproduzierbarer Weise abgesenkt.

In einer weiteren ganz besonders bevorzugten Ausführungsform ist das erfindungsgemäße Verfahren zur oxidativen Farbveränderung von menschlichen Haaren dadurch gekennzeichnet, dass dass die Komponente (K2) - bezogen auf das Gesamtgewicht der Komponente (K2) - 1,0 bis 8,0 Gew.-%, bevorzugt 1,5 bis 6,0 Gew.-% und besonders bevorzugt 1,9 bis 4,5 Gew.-% Ammoniak (berechnet als (NH3)) enthält.

Ammoniak wird üblicherweise als eine 25 %ige wässrige Lösung eingesetzt. Alle Mengenangaben in Gew.-% beziehen sich auf die in der Komponente (K2) enthaltenen Gesamt an Ammoniak (berechnet als NH3), die zum Gesamtgewicht der Färbezubereitung (K2) in Relation gesetzt wird.

Aufgrund ihres hohen Gehaltes an Alkalisierungsmittel (insbesondere Ammoniak) besitzt die Färbezubereitung (d.h. die zweite Komponente (K2)) einen entsprechend hohen pH-Wert, der im alkalischen Bereich oberhalb von 10,0 liegt.

In einer weiteren ganz besonders bevorzugten Ausführungsform ist das erfindungsgemäße Verfahren zur oxidativen Farbveränderung von menschlichen Haaren dadurch gekennzeichnet, dass die Komponente (K2) Wasser enthält und dass der pH-Wert der Komponente (K2) bei einem Wert von 10,0 bis 10,8, bevorzugt von 10,1 bis 10,6 und ganz besonders bevorzugt von 10,1 bis 10,5 liegt.

Durch das Vermischen mit der besonders sauren Oxidationsmittelzubereitung (K1) soll dieser pH-Wert im anwendungsbereiten Mittel definiert auf einen Wert abgesenkt werden, bei zwar immer noch ein intensives Färberesultat erzielt werden kann, weitere Haarschädigungen jedoch soweit wie möglich vermieden werden können. Es hat sich herausgestellt, dass die Haarschädigung signifikant reduziert werden kann, wenn der pH-Wert des anwendungsbereiten Färbemittels (d.h. der Mischung (M1)) auf einen pH-Wert unterhalb von 10 und oberhalb von 9 abgesenkt wird.

In einer weiteren ganz besonders bevorzugten Ausführungsform ist das erfindungsgemäße Verfahren zur oxidativen Farbveränderung von menschlichen Haaren dadurch gekennzeichnet, dass
- der pH-Wert der Mischung (M1) bei einem Wert von 9,0 bis 10,0, bevorzugt von 9,5 bis 9,9 und ganz besonders bevorzugt von 9,65 bis 9,85 liegt.

In allen bislang beschriebenen Ausführungsformen wurde dem Friseur die erfindungsgemäße Oxidationsmittelzubereitung (entsprechend der Komponente (K1)) bereits als fertige Zubereitung zur Verfügung gestellt. In einer weiteren Ausführungsform ist es jedoch ebenfalls möglich, dass der Friseur die Oxidationsmittelzubereitung (K1) durch Ansäuern einer handelsüblichen, aus dem Stand der Technik bekannten Oxidationsmittelzubereitung selber herstellt.

Der pH-Wert der handelsüblichen Oxidationsmittelzubereitungen liegt in der Regel bei ca. 3,5. Zu dieser handelsüblichen Oxidationsmittelzubereitung kann der Friseur nun ein saures Konzentrat, welches eine oder mehrere der zuvor beschriebenen Säuren aus der Gruppe (A) (bzw. aus den Gruppen (A) und (C)) enthält, hinzufügen und damit den pH-Wert der Oxidationsmittelzubereitung auf den erfindungsgemäßen Bereich von 0,5 bis 3,0, insbesondere von 0,5 bis 1,8, absenken.

Diese Vorgehensweise hat den ganz besonderen Vorteil, dass für die Herstellung einer Level 2 Coloration aus einer Level 3 Coloration sowohl die für das Level 3 Produkt üblich Oxidationsmittelzubereitung als auch die Farbcreme verwendet werden kann, und der Friseur zusätzlich lediglich ein Säure-Konzentrat kaufen und lagern muss.

Ein weiterer Gegenstand der vorliegenden Erfindung ist daher ein Verfahren zur oxidativen Farbveränderung von menschlichen Haaren, umfassend die folgenden Schritte in der angegebenen Reihenfolge
A) Vermischen einer ersten Komponente (Ka) mit einer zweiten Komponente (Kb) unter Erhalt einer ersten Mischung (Ma),
B) Vermischen der ersten Mischung (Ma) mit einer dritten Komponente (Kc) unter Erhalt einer zweiten Mischung (Mb),
C) Applizieren der zweiten Mischung (Mb) auf die Haare,
D) Einwirkenlassen der Mischung (Mb) für einen Zeitraum von 30 Sekunden bis 45 Minuten,
E) Ausspülen der Mischung (Mb) aus den Haaren,
wobei
- die erste Komponente (Ka) eine Oxidationsmittelzubereitung ist, die Wasserstoffperoxid enthält,
- die zweite Komponente (Kb) ist ein Oxidationsmittel-Verdünner, hier also Äpfelsäure,
- die Mischung (M1) eine Oxidationsmittelzubereitung ist, wie sie bei Beschreibung des ersten Erfindungsgegenstands im Detail offenbart wurde, und
- die dritte Komponente (Kc) eine Färbezubereitung ist, die mindestens ein Oxidationsfarbstoffvorprodukt und/oder mindestens ein Alkalisierungsmittel enthält.

Im ersten Schritt A) dieses erfindungsgemäßen Verfahrens wird die (aus dem Stand der Technik bekannte) Oxidationsmittelzubereitung (Ka) mit einem Oxidationsmittelverdünner (Kb) vermischt. Die Vermischung von (Ka) und (Kb) kann beispielsweise durch Verrühren oder Verschütteln erfolgen,

Die Oxidationsmittelzubereitung (Ka) enthält in einem wässrigen kosmetischen Träger Wasserstoffperoxid und besitzt einen pH-Wert, der bei ca. 3,5 liegt.

Der Oxidationsmittelverdünner (Kb) ist Äpfelsäure.

Durch Vermischen der Oxidationsmittelzubereitung (Ka) mit dem Oxidationsmittel-Verdünner (Kb) wird eine erste Mischung (Ma) erhalten. Bei dieser ersten Mischung (Ma) handelt es sich um die erfindungsgemäße Oxidationsmittelzubereitung, wie sie bei Beschreibung des ersten Erfindungsgegenstandes im Detail offenbart ist.

Wenn die Komponenten (Ka) und (Ka) vollständig miteinander vermischt sind, d.h. wenn eine homogene Mischung (Ma) vorliegt, wird diese Mischung im nächsten Schritt B) wiederum mit der dritten Komponente (Kc) vermischt.

Bei der dritten Komponente (Kc) handelt es sich wieder um die üblicherweise vom Friseur verwendete Level 3 Farbcreme, die mindestens ein Oxidationsfarbstoffvorprodukt und/oder mindestens ein Alkalisierungsmittel enthält. Bevorzugt enthält die zweite Komponente (Kc) mindestens ein Oxidationsfarbstoffvorprodukt vom Entwicklertyp und mindestens ein Oxidationsfarbstoffvorprodukt vom Kupplertyp.

Die Vermischung von (Ma) und (Kc) kann beispielsweise durch Verrühren oder Verschütteln erfolgen, hierbei resultiert die zweite Mischung (Mb). Die zweite Mischung (Mb) wird dann auf die Haare appliziert.

Die Schritte A) bis E) sind die Schritte eines einzigen Färbeverfahrens, d.h. alle Schritte werden erfindungsgemäß während eines Färbevorgangs, d.h. innerhalb eines Zeitraumes von maximal 6 Stunden, bevorzugt innerhalb eines Zeitraums von maximal 3 Stunden, durchgeführt.

Im erfindungsgemäßen Verfahren ist auch die Reihenfolge der Schritte festgelegt und findet in der Reihenfolge A), gefolgt von B), gefolgt von C), gefolgt von D) gefolgt von E) statt.

Als besonders vorteilhaft hat sich herausgestellt, dass die Verdünnung des Oxidationsmittels, d.h. die Herstellung der ersten Mischung (Ma) aus der handelsüblichen Oxidationsmittelzubereitung (Ka) und dem Oxidationsmittel-Verdünner (Kb) durchgeführt werden konnte, ohne dass die durch die Vermischung mit den Oxidationsfarbstoffvorprodukten bereits die Farbstoffbildung initiiert wurde. Auf diese Weise konnte ein gleichmäßigeres und reproduzierbareres Farbergebnis erzielt werden.

Aus diesem Grund ist die Herstellung dieser ersten Mischung (Ma) aus (Ka) und (Kb) in Schritt A) ein ganz zentraler und wesentlicher Schritt dieses erfindungsgemäßen Verfahrens.

Wurde hingegen zuerst ein übliches oxidatives Färbemittel durch Vermischen der Komponenten (Ka) und (Kc) hergestellt und dieses danach mit der (Kb) verdünnt, so liefen der Farbstoffbildungsprozess und die Ansäuerung parallel ab. Bei diesem - nicht erfindungsgemäßen - Verfahren wurde die Intensität der finalen Haarfärbung somit wesentlich von der Dauer mitbeeinflusst, die für das Untermischen der Komponente (Kb) benötigt wurde. Die Färberesultate, die über dieses nicht erfindungsgemäße Verfahren erzielt wurden, waren somit weniger reproduzierbar und wichen von Färbevorgang zu Färbevorgang stark ab.

Die Komponente (Kc) entspricht der Komponente (K2) der zuvor beschriebenen Verfahrens. Für bevorzugte Ausführungsformen der Komponente (Kc) gilt daher mutatis mutandis das für die Komponente (K2) gesagte.

Bevorzugt werden die Komponenten (Ka) und (Kb) im erfindungsgemäßen Verfahren in bestimmten Mengenbereichen miteinander vermischt. Die erste Komponente (Ka) und die zweite Komponente (Kb) können beispielsweise in einem Mengenverhältnis von 3:1 bis 1:3, bevorzugt von 2:1 bis 2:1, miteinander vermischt werden.

Bevorzugt werden die Komponenten (Ma) und (Kc) im erfindungsgemäßen Verfahren in bestimmten Mengenbereichen miteinander vermischt. Die erste Mischung (Ma) und die dritte Komponente (Mc) können beispielsweise in einem Mengenverhältnis von 3:1 bis 1:3, bevorzugt von 2:1 bis 2:1, miteinander vermischt werden.

In einer weiteren ganz besonders bevorzugten Ausführungsform ist das Verfahren zum Färben von menschlichen Haaren, dadurch gekennzeichnet, dass
A) die erste Komponente (Ka) und die zweite Komponente (Kb) in einem Mengenverhältnis von 3:1 bis 1:3, bevorzugt von 2:1 bis 2:1, miteinander vermischt werden und
B) die erste Mischung (Ma) und die dritte Komponente (Kc) in einem Mengenverhältnis von 3:1 bis 1:3, bevorzugt von 2:1 bis 1:2, miteinander vermischt werden

Wie bereits zuvor beschrieben, soll das erfindungsgemäße Verfahren den Friseur auch in die Lage versetzen, besonders geschädigte Partien des Haares gezielt mit einem weniger schädigenden oxidativen Färbemittel zu behandeln.

In geschädigtem Haar ist die Cuticula, die Schuppenschicht des Haares, in einem mehr oder weniger starken Ausmaß zerstört. Dies führt dazu, dass auf geschädigtem Haar generell ein stärkerer Farbaufzug stattfindet. Wenn Ansatz und Spitzen mit demselben Färbemittel gefärbt werden, besteht bei stärker geschädigtem Haar daher immer die Gefahr eines ungleichmäßigen Farbergebnisses.

Abhängig vom Schädigungsgrad der jeweiligen Haarpartie kann der Friseur somit bevorzugt wählen, ob er die Mischung (Mb) in Schritt C) des Verfahrens auf die Haare im gesamten zu färbenden Bereich appliziert, oder ab der die Mischung (M2) gezielt nur auf bestimmte Haarpartien aufträgt.

In einer weiteren ganz besonders bevorzugten Ausführungsform ist das Verfahren zum Färben von menschlichen Haaren, dadurch gekennzeichnet, dass C) die Mischung (Mb) auf die Haare im Ansatzbereich oder im Bereich der Haarspitzen appliziert wird.

Weiterhin kann der Friseur abhängig vom Schädigungsgrad auch die Einwirkzeit der Mischung (Mb) auf bestimmte Haarpartien unterschiedlich wählen.

Nach dem Auftragen auf die Haare wird die Mischung (Mb) in Schritt D) für einen Zeitraum von 30 Sekunden bis 45 Minuten auf die Haare einwirken gelassen. Hierbei ist es möglich und erfindungsgemäß, die Mischung (Mb) für einen bestimmten Zeitraum auf allen Partien des Haares zu belassen. In einer weiteren Ausführungsform ist es jedoch ebenfalls möglich, die Einwirkdauer für bestimmte Haarpartien verschieden zu wählen, so dass beispielsweise die Einwirkdauer im Bereich das Haaransatzes länger ist als die Einwirkdauer im Bereich der geschädigten Spitzen.

In einer weiteren ganz besonders bevorzugten Ausführungsform ist das Verfahren zum Färben von menschlichen Haaren, dadurch gekennzeichnet, dass
D1) die Mischung (Mb) für einen Zeitraum von 30 Sekunden bis 45 Minuten auf die Haare im Bereich des Haaransatzes oder der Haarspitzen appliziert wird und
D2) die Mischung (Mb) für einen Zeitraum von 30 Sekunden bis 45 Minuten auf die Haare in dem Bereich appliziert wird, der in Schritt D1) noch nicht behandelt wurde,
wobei die Applikationsdauern der Schritte D1) und D2) sich um mindestens 5 Minuten, bevorzugt mindestens 10 Minuten unterscheiden.

Sind beispielsweise die Haare im gesamten Bereich - außer direkt am Ansatz - sehr stark geschädigt, so kann der Friseur
D1) die Mischung (Mb) für einen Zeitraum von 35 bis 45 Minuten auf die Haare im Bereich des Haaransatzes applizieren und
D2) die Mischung (Mb) nur für einen Zeitraum von 25 bis 30 Minuten auf die Haare im Bereich außerhalb des Ansatzes applizieren.

Sind lediglich die Spitzen stark geschädigt, so kann der Friseur
D1) die Mischung (Mb) für einen Zeitraum von 15 bis 25 Minuten auf die Haare im Bereich der Haarspitzen applizieren und
D2) die Mischung (Mb) nur für einen Zeitraum von 25 bis 30 Minuten auf die Haare im Bereich außerhalb der Haarspitzen applizieren.

Unter dem Haaransatz werden erfindungsgemäß die direkt an der Kopfhaut befindliche Haarpartie (die ersten 0 bis 5 cm des Haares) verstanden.

Unter dem Bereich der Haarspitzen werden - abhängig von der Haarlänge - die letzten 5 bis 10 cm des Haares verstanden).

Die zuvor beschriebenen Komponenten (K1), (K2) (Ka), (Kb) und (Kc) können ferner auch weitere Wirk-, Hilfs- und Zusatzstoffe enthalten, wie beispielsweise Fettbestandteile, Tenside, nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Betreffend weitere Ausführungsformen der erfindungsgemäßen Verfahren gilt mutatis mutandis das zu den erfindungsgemäßen Oxidationsmittelzubereitungen gesagte.
- alle nicht erfindungsgemäss und daher nur zum Vergleich Es wurden die folgenden Formulierungen hergestellt - alle Angaben erfolgen, sofern nicht anders angegeben, in Gewichtsprozent.

### 1. Oxidationsmittelzubereitung (Komponente (K1))

| | (K1) V | (K1)E |
|---|---|---|
| Natriumbenzoat | 0,1 | 0,1 |
| Dipicolinsäure | 0,1 | 0,1 |
| Dinatriumpyrophosphat | 0,1 | 0,1 |
| 1,2-Propandiol | 1,0 | 1,0 |
| Etidronsäure (1-Hydroxyehtan-1,1-diphosphonsäure) | 0,2 | 0,2 |
| Paraffinum Liquidum | 0,3 | 0,3 |
| Steartrimoniumchlorid | 0,4 | 0,4 |
| Cetearylalkohol | 3,4 | 3,4 |
| Ceteareth-20 | 1,0 | 1,0 |
| Phosphorsäure | --- | 1,0 |
| Wasserstoffperoxid | 6,1 | 6,1 |
| Wasser | ad 100 | ad 100 |
| pH-Wert | pH = 3,5 | pH = 1,42 |

### 2. Färbezubereitung (Komponente (K2))

| | |
|---|---|
| | |
| Polyacrylsäure (Ammoniumsalz) 0,5 %ige wässrige Lösung | 15,0 |
| Decyloleat | 2,1 |
| Natriumcetearylsulfat | 1,3 |
| Cetearylalkohol | 14,9 |
| Glycerylstearat | 5,4 |
| Linoleamidopropyl PG-dimoniumchlorid Phosphat | 0,05 |
| EDTA | 0,8 |
| Monoethanolamin | 0,4 |
| Ammoniak (25 %ige wässrige Lösung) | 8,0 |
| Ascorbinsäure | 0,1 |
| Natriumdithionit | 0,1 |
| L-SerinO | 0,3 |
| Polyquaternium-2 | 0,1 |
| p-Toluylendiamin, sulfat | 0,8 |
| Resorcin | 0,2 |
| m-Aminophenol | 0,04 |
| 4-Chlorresorcin | 0,2 |
| 2-Amino-4-[(2-hydroxyethyl)amino]-anisol | 0,02 |
| Wasser | ad 100 |

### 3. Anwendung

Die Oxidationsmittelzubereitung (K1) wurde jeweils im Mengenverhältnis 1:1 mit der Färbezubereitung (K2) vermischt.

Es wurden die folgenden Resultate erhalten

| | (K1) V + (K2) Vergleich | (K1) E + (K2) Erfindung |
|---|---|---|
| pH-Wert | 10,05 | 9,79 |

### 4. Formulierungsbeispiele

| Oxidationsmittelzubereitung (Komponente (K1)) | |
|---|---|
| Natriumbenzoat | 0,05 |
| Dipicolinsäure | 0,13 |
| Dinatriumpyrophosphat | 0,13 |
| 1,2-Propandiol | 1,00 |
| Etidronsäure (1-Hydroxyehtan-1,1-diphosphonsäure) | 0,20 |
| Paraffinum Liquidum | 20,0 |
| Steartrimoniumchlorid | 0,39 |
| Cetearylalkohol | 4,70 |
| Ceteareth-20 | 2,80 |
| Glycerylstearat | 1,35 |
| Wasserstoffperoxid | 3,1 |
| Weinsäure | ad pH 2,1 |
| Wasser | ad 100 |

## Patentansprüche

1. Oxidationsmittelzubereitung zur oxidativen Farbveränderung von keratinischen Fasern, insbesondere menschlichen Haaren, enthaltend in einem wässrigen kosmetischen Träger
(A) Wasserstoffperoxid und
(B) Äpfelsäure, wobei die Oxidationsmittelzubereitung einen pH-Wert von 0,5 bis 3,0 besitzt.

2. Oxidationsmittelzubereitung nach Anspruch 1, **dadurch gekennzeichnet, dass** sie - bezogen auf ihr Gesamtgewicht -
(A) Wasserstoffperoxid in einer Menge von 0,5 bis 12,5 Gew.-%, bevorzugt von 1,0 bis 9,0 Gew.-%, weiter bevorzugt von 1,5 bis 6,5 Gew.-% und ganz besonders bevorzugt von 2,0 bis 4,5 Gew-% enthält.

3. Oxidationsmittelzubereitung nach einem der Ansprüche 1 bis 2, **dadurch gekennzeichnet, dass** sie einen pH-Wert von 0,5 bis 2,6, bevorzugt von 0,5 bis 2,4, weiter bevorzugt von 0,5 bis 2,2, noch weiter bevorzugt von 0,5 bis 2,0, noch weiter bevorzugt von 0,5 bis 1,8 und ganz besonders bevorzugt von 0,5 bis 1,6 besitzt.

4. Oxidationsmittelzubereitung nach einem der Ansprüche 1 bis 3, **dadurch gekennzeichnet, dass** sie zusätzlich
(C) mindestens eine Säure aus der Gruppe aus Etidronsäure, Benzoesäure, Pyridin-2,6-dicarbonsäure, Phthalsäure und/oder Salicylsäure enthält.

5. Oxidationsmittelzubereitung nach einem der Ansprüche 1 bis 4, **dadurch gekennzeichnet, dass** sie - bezogen auf ihr Gesamtgewicht - eine Säure aus der Gruppe (B) in einer Gesamtmenge von 0,5 bis 15,0 Gew.-%, bevorzugt von 0,5 bis 10,0 Gew.-%, weiter bevorzugt von 0,5 bis 8,0 Gew.-% und ganz besonders bevorzugt von 1,0 bis 6,0 Gew.-% enthält.

6. Oxidationsmittelzubereitung nach einem der Ansprüche 1 bis 5, **dadurch gekennzeichnet, dass** sie - bezogen auf ihr Gesamtgewicht - einen oder mehrere Fettbestandteile in einer Gesamtmenge 1,0 bis 10,0 Gew.-%, bevorzugt von 1,5 bis 7,5 Gew.-%, weiter bevorzugt von 2,0 bis 7,0 Gew.-% und ganz besonders bevorzugt von 2,5 bis 6,5 Gew.-% enthält.

7. Verfahren zur oxidativen Farbveränderung von menschlichen Haaren, umfassend die folgenden Schritte in der angegebenen Reihenfolge
A) Vermischen einer ersten Komponente (K1) mit einer zweiten Komponente (K2) unter Erhalt einer Mischung (M1),
B) Applizieren der Mischung (M1) auf die Haare,
C) Einwirkenlassen der Mischung (M1) für einen Zeitraum von 30 Sekunden bis 45 Minuten,
D) Ausspülen der Mischung (M1) aus den Haaren,
wobei
- die erste Komponente (K1) eine Oxidationsmittelzubereitung ist, wie sie in einem der Ansprüche 1 bis 6 beschrieben ist, und
- die zweite Komponente (K2) eine Färbezubereitung ist, die mindestens ein Oxidationsfarbstoffvorprodukt und/oder mindestens ein Alkalisierungsmittel enthält.

8. Verfahren nach Anspruch 7, **dadurch gekennzeichnet, dass**
- die erste Komponente (K1) und die zweite Komponente (K2) in einem Gewichtsverhältnis von 3:1 bis 1:3, bevorzugt von 2:1 bis 1:2 und besonders bevorzugt von 1,5:1 bis 1:1,5 miteinander vermischt werden.

9. Verfahren nach einem der Ansprüche 7 bis 8, **dadurch gekennzeichnet, dass** die zweite Komponente (K2) mindestens ein Oxidationsfarbstoffvorprodukt aus der Gruppe aus p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4-Aminophenol, 4-Amino-3-methylphenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin und/oder deren physiologisch verträglichen Salzen enthält.

10. Verfahren nach einem der Ansprüche 7 bis 9, **dadurch gekennzeichnet, dass** die Komponente (K2) - bezogen auf das Gesamtgewicht der Komponente (K2) - 1,0 bis 8,0 Gew.-%, bevorzugt 1,5 bis 6,0 Gew.-% und besonders bevorzugt 1,9 bis 4,5 Gew.-% Ammoniak (berechnet als (NH₃)) enthält.

11. Verfahren nach einem der Ansprüche 7 bis 10, **dadurch gekennzeichnet, dass**
- die Komponente (K2) Wasser enthält und dass der pH-Wert der Komponente (K2) bei einem Wert von 10,0 bis 10,8, bevorzugt von 10,1 bis 10,6 und ganz besonders bevorzugt von 10,1 bis 10,5 liegt.

12. Verfahren nach einem der Ansprüche 7 bis 11, **dadurch gekennzeichnet, dass**
- der pH-Wert der Mischung (M1) bei einem Wert von 9,0 bis 10,0, bevorzugt von 9,5 bis 9,9 und ganz besonders bevorzugt von 9,65 bis 9,85 liegt

13. Verfahren zur oxidativen Farbveränderung von menschlichen Haaren, umfassend die folgenden Schritte in der angegebenen Reihenfolge
A) Vermischen einer ersten Komponente (Ka) mit einer zweiten Komponente (Kb) unter Erhalt einer ersten Mischung (Ma),
B) Vermischen der ersten Mischung (Ma) mit einer dritten Komponente (Kc) unter Erhalt einer zweiten Mischung (Mb),
C) Applizieren der zweiten Mischung (Mb) auf die Haare,
D) Einwirkenlassen der Mischung (Mb) für einen Zeitraum von 30 Sekunden bis 45 Minuten,
E) Ausspülen der Mischung (Mb) aus den Haaren,
wobei
- die erste Komponente (Ka) eine Oxidationsmittelzubereitung ist, die Wasserstoffperoxid enthält,
- die zweite Komponente (Kb) ein Oxidationsmittel-Verdünner ist, der enthält,
- die Mischung (M1) eine Oxidationsmittelzubereitung ist, wie sie in einem der Ansprüche 1 bis 6 beschrieben ist, und
- die dritte Komponente (Kc) eine Färbezubereitung ist, die mindestens ein Oxidationsfarbstoffvorprodukt und/oder mindestens ein Alkalisierungsmittel enthält.

14. Verfahren nach Anspruch 13, **dadurch gekennzeichnet, dass**
A) die erste Komponente (Ka) und die zweite Komponente (Kb) in einem Mengenverhältnis von 3:1 bis 1:3, bevorzugt von 2:1 bis 2:1, miteinander vermischt werden und
B) die erste Mischung (Ma) und die dritte Komponente (Kc) in einem Mengenverhältnis von 3:1 bis 1:3, bevorzugt von 2:1 bis 1:2, miteinander vermischt werden

## Claims

1. An oxidizing agent preparation for oxidatively changing the color of keratin fibers, in particular human hair, containing in an aqueous cosmetic carrier
(A) hydrogen peroxide, and
(B) malic acid,
wherein the oxidizing agent preparation has a pH of 0.5 to 3.0.

2. The oxidizing agent preparation according to claim 1, **characterized in that** it contains, based on its total weight,
(A) hydrogen peroxide in an amount of 0.5 to 12.5 wt.%, preferably of 1.0 to 9.0 wt.%, more preferably of 1.5 to 6.5 wt.% and very particularly preferably of 2.0 to 4.5 wt.%.

3. The oxidizing agent preparation according to one of claims 1 to 2, **characterized in that** it has a pH of 0.5 to 2.6, preferably of 0.5 to 2.4, more preferably of 0.5 to 2.2, even more preferably of 0.5 to 2.0, yet more preferably of 0.5 to 1.8 and very particularly preferably of 0.5 to 1.6.

4. The oxidizing agent preparation according to one of claims 1 to 3, **characterized in that** it additionally contains
(C) at least one acid from the group of etidronic acid, benzoic acid, pyridine-2,6-dicarboxylic acid, phthalic acid and/or salicylic acid.

5. The oxidizing agent preparation according to one of claims 1 to 4, **characterized in that** it contains, based on its total weight, an acid from the group (B) in a total amount of 0.5 to 15.0 wt.%, preferably of 0.5 to 10.0 wt.%, more preferably of 0.5 to 8.0 wt.% and very particularly preferably of 1.0 to 6.0 wt.%.

6. The oxidizing agent preparation according to one of claims 1 to 5, **characterized in that** it contains, based on its total weight, one or more fat constituents in a total amount of 1.0 to 10.0 wt.%, preferably of 1.5 to 7.5 wt.%, more preferably of 2.0 to 7.0 wt.% and very particularly preferably of 2.5 to 6.5 wt.%.

7. A method for oxidatively changing the color of human hair, comprising the following steps in the sequence indicated:
A) mixing a first component (K1) with a second component (K2) to obtain a mixture (M1),
B) applying the mixture (M1) to the hair,
C) allowing the mixture (M1) to act for a period of 30 seconds to 45 minutes,
D) rinsing the mixture (M1) out of the hair,
wherein
- the first component (K1) is an oxidizing agent preparation as described in one of claims 1 to 6, and
- the second component (K2) is a dye preparation which contains at least one oxidation dye precursor and/or at least one alkalizing agent.

8. The method according to claim 7, **characterized in that**
- the first component (K1) and the second component (K2) are mixed together in a weight ratio of 3:1 to 1:3, preferably of 2:1 to 1:2, and particularly preferably of 1.5:1 to 1:1.5.

9. The method according to one of claims 7 to 8, **characterized in that** the second component (K2) contains at least one oxidation dye precursor from the group of p-toluenediamine, 2-(2-hydroxyethyl)-p-phenylenediamine, N,N-bis-(2-hydroxyethyl)-p-phenylenediamine, 2-methoxymethyl-p-phenylenediamine, N-(4-amino-3-methylphenyl)-N-[3-(1H-imidazol-1-yl)propyl]amine, bis-(2-hydroxy-5-aminophenyl)methane, 4-aminophenol, 4-amino-3-methylphenol, 4,5-diamino-1-(2-hydroxyethyl)pyrazole, 2,4,5,6-tetraaminopyrimidine, 4-hydroxy-2,5,6-triaminopyrimidine, 2-hydroxy-4,5,6-triaminopyrimidine and/or the physiologically acceptable salts thereof.

10. The method according to one of claims 7 to 9, **characterized in that** the component (K2) contains, based on the total weight of the component (K2), 1.0 to 8.0 wt.%, preferably 1.5 to 6.0 wt.%, and particularly preferably 1.9 to 4.5 wt.%, of ammonia (calculated as (NH₃)).

11. The method according to one of claims 7 to 10, **characterized in that**
- the component (K2) contains water and **in that** the pH of the component (K2) has a value of 10.0 to 10.8, preferably of 10.1 to 10.6 and very particularly preferably of 10.1 to 10.5.

12. The method according to one of claims 7 to 11, **characterized in that**
- the pH of the mixture (M1) has a value of 9.0 to 10.0, preferably of 9.5 to 9.9 and very particularly preferably of 9.65 to 9.85.

13. A method for oxidatively changing the color of human hair, comprising the following steps in the sequence indicated:
A) mixing a first component (Ka) with a second component (Kb) to obtain a first mixture (Ma),
B) mixing the first mixture (Ma) with a third component (Kc) to obtain a second mixture (Mb),
C) applying the second mixture (Mb) to the hair,
D) allowing the mixture (Mb) to act for a period of 30 seconds to 45 minutes,
E) rinsing the mixture (Mb) from the hair,
wherein
- the first component (Ka) is an oxidizing agent preparation which contains hydrogen peroxide,
- the second component (Kb) is an oxidizing agent thinner which contains malic acid,
- the mixture (M1) is an oxidizing agent preparation as described in one of claims 1 to 6, and
- the third component (Kc) is a dye preparation which contains at least one oxidation dye precursor and/or at least one alkalizing agent.

14. The method according to claim 13, **characterized in that**
A) the first component (Ka) and the second component (Kb) are mixed together in a quantity ratio of 3:1 to 1:3, preferably of 2:1 to 2:1, and
B) the first mixture (Ma) and the third component (Kc) are mixed together in a quantity ratio of 3:1 to 1:3, preferably of 2:1 to 1:2.

## Revendications

1. Préparation d'oxydant pour le changement de couleur par oxydation de fibres kératiniques, en particulier de cheveux humains, contenant dans un support cosmétique aqueux
(A) du peroxyde d'hydrogène et
(B) de l'acide malique,
la préparation d'oxydant présentant un pH de 0,5 à 3,0.

2. Préparation d'oxydant selon la revendication 1, **caractérisée en ce qu'**elle contient - par rapport à son poids total -
(A) du peroxyde d'hydrogène en une quantité de 0,5 à 12,5 % en poids, de préférence de 1,0 à 9,0 % en poids, de manière davantage préférée de 1,5 à 6,5 % en poids et de manière tout particulièrement préférée de 2,0 à 4,5 % en poids.

3. Préparation d'oxydant selon l'une des revendications 1 et 2, **caractérisée en ce qu'**elle présente un pH de 0,5 à 2,6, de préférence de 0,5 à 2,4, de manière davantage préférée de 0,5 à 2,2, de manière encore davantage préférée de 0,5 à 2,0, de manière encore plus préférée de 0,5 à 1,8 et de manière tout particulièrement préférée de 0,5 à 1,6.

4. Préparation d'oxydant selon l'une des revendications 1 à 3, **caractérisée en ce qu'**elle contient en plus
(C) au moins un acide du groupe constitué par l'acide étidronique, l'acide benzoïque, l'acide pyridine-2,6-dicarboxylique, l'acide phtalique et/ou l'acide salicylique.

5. Préparation d'oxydant selon l'une des revendications 1 à 4, **caractérisée en ce qu'**elle contient - par rapport à son poids total - un acide du groupe (B) en une quantité totale de 0,5 à 15,0 % en poids, de préférence de 0,5 à 10,0 % en poids, de manière davantage préférée de 0,5 à 8,0 % en poids et de manière tout particulièrement préférée de 1,0 à 6,0 % en poids.

6. Préparation d'oxydant selon l'une des revendications 1 à 5, **caractérisée en ce qu'**elle contient - par rapport à son poids total - un ou plusieurs composants gras en une quantité totale de 1,0 à 10,0 % en poids, de préférence de 1,5 à 7,5 % en poids, de manière davantage préférée de 2,0 à 7,0 % en poids et de manière tout particulièrement préférée de 2,5 à 6,5 % en poids.

7. Procédé de changement de couleur par oxydation de cheveux humains, comprenant les étapes suivantes dans l'ordre indiqué
A) mélange d'un premier constituant (K1) avec un second constituant (K2) pour obtenir un mélange (M1),
B) application du mélange (M1) sur les cheveux,
C) pose du mélange (M1) pendant une durée de 30 secondes à 45 minutes,
D) élimination par rinçage du mélange (M1) présent sur les cheveux,
- le premier constituant (K1) étant une préparation d'oxydant telle que décrite dans l'une des revendications 1 à 6,
- le second constituant (K2) étant une préparation colorante qui contient au moins un précurseur de colorant par oxydation et/ou au moins un agent d'alcalinisation.

8. Procédé selon la revendication 7, **caractérisé en ce que**
- le premier constituant (K1) et le second constituant (K2) sont mélangés l'un avec l'autre selon un rapport en poids de 3:1 à 1:3, de préférence de 2:1 à 1:2 et de manière particulièrement préférée de 1,5:1 à 1:1,5.

9. Procédé selon l'une des revendications 7 et 8, **caractérisé en ce que** le second constituant (K2) contient au moins un précurseur de colorant par oxydation choisi dans le groupe constitué par la p-toluylènediamine, la 2-(2-hydroxyéthyl)-p-phénylènediamine, la N,N-bis-(2-hydroxyéthyl)-p-phénylènediamine, la 2-méthoxyméthyl-p-phénylènediamine, la N-(4-amino-3-méthylphényl)-N-[3-(1H-imidazol-1-yl)propyl]amine, le bis-(2-hydroxy-5-aminophényl)méthane, le 4-aminophénol, le 4-amino-3-méthylphénol, le 4,5-diamino-1-(2-hydroxyéthyl)pyrazole, la 2,4,5,6-tétraaminopyrimidine, la 4-hydroxy-2,5,6-triaminopyrimidine, la 2-hydroxy-4,5,6-triaminopyrimidine et/ou leurs sels physiologiquement acceptables.

10. Procédé selon l'une des revendications 7 à 9, **caractérisé en ce que** le constituant (K2) contient - par rapport au poids total du constituant (K2) - 1,0 à 8,0 % en poids, de préférence 1,5 à 6,0 % en poids et de manière particulièrement préférée 1,9 à 4,5 % en poids d'ammoniac (calculé en tant que (NH₃)).

11. Procédé selon l'une des revendications 7 à 10, **caractérisé en ce que**
- le constituant (K2) contient de l'eau et **en ce que** le pH du constituant (K2) est égal à une valeur comprise entre 10,0 et 10,8, de préférence 10,1 et 10,6 et de manière tout particulièrement préférée 10,1 et 10,5.

12. Procédé selon l'une des revendications 7 à 11, **caractérisé en ce que**
- le pH du mélange (M1) est égal à une valeur comprise entre 9,0 et 10,0, de préférence 9,5 et 9,9 et de manière tout particulièrement préférée 9,65 et 9,85.

13. Procédé de changement de couleur par oxydation de cheveux humains, comprenant les étapes suivantes dans l'ordre indiqué
A) mélange d'un premier constituant (Ka) avec un deuxième constituant (Kb) pour obtenir un premier mélange (Ma),
B) mélange du premier mélange (Ma) avec un troisième constituant (Kc) pour obtenir un second mélange (Mb),
C) application du second mélange (Mb) sur les cheveux,
D) pose du mélange (Mb) pendant une durée de 30 secondes à 45 minutes,
E) élimination par rinçage du mélange (Mb) présent sur les cheveux,
- le premier constituant (Ka) étant une préparation d'oxydant, qui contient du peroxyde d'hydrogène.
- le deuxième constituant (Kb) étant un agent d'oxydation diluant, qui contient de l'acide malique,
- le mélange (M1) étant une préparation d'oxydant telle que décrite dans l'une des revendications 1 à 6, et
- le troisième constituant (Kc) étant une préparation colorante qui contient au moins un précurseur de colorant par oxydation et/ou au moins un agent d'alcalinisation.

14. Procédé selon la revendication 13, **caractérisé en ce que**
A) le premier constituant (Ka) et le deuxième constituant (Kb) sont mélangés l'un avec l'autre selon un rapport en poids de 3:1 à 1:3, de préférence de 2:1 à 2:1, et
B) le premier mélange (Ma) et le troisième constituant (Kc) sont mélangés l'un avec l'autre selon un rapport en poids de 3:1 à 1:3, de préférence de 2:1 à 1:2.
